# EUROPEAN PATENT SPECIFICATION

(11) **EP 0 468 435 B1**
(45) Date of publication and mention of the grant of the patent: **16.10.1996**
(21) Application number: 91112312.3
(22) Date of filing: 23.07.1991
(51) Int. Cl.: A61K 6/06, A61K 6/027

(54) **Dental ceramic composition for titanium prostheses**
Dentalkeramikzusammensetzung für Titanium-Prothesen
Composition de céramique dentaire pour les prothèses en titane

(30) Priority: 24.07.1990 US 557260
(43) Date of publication of application: 29.01.1992
(73) Proprietor: DENTSLPLY GmbH, D-63264 Dreieich (DE)
(72) Inventor: Weissbach, Klaus, W-6550 Bad Kreuznach (DE); Krumbholz, Klaus, W-6070 Langen (DE); Janda, Ralf, W-6380 Bad Homburg (DE)
(74) Representative: Wächtershäuser, Günter, Prof. Dr.

(56) References cited:
- EP-A- 0 119 063
- EP-A- 0 328 772
- FR-A- 2 305 169
- FR-A- 2 391 175
- US-A- 4 198 244
- CHEMICAL ABSTRACTS, vol. 111, no. 6, 7 August 1989, Columbus, Ohio, US; abstract no. 45348W,
- PATENT ABSTRACTS OF JAPAN vol. 13, no. 523 (C-657)(3871) 21 November 1989

## Description

### BACKGROUND OF THE INVENTION

This invention relates to a dental ceramic composition that can be fused to dental prostheses including crowns, bridges, or prostheses parts made of titanium or titanium alloys. The response is a strong and permanent bond between the dental ceramic and the titanium or titanium alloys.

Chem. Abs., Vol 111 (1989) 45348w discloses a ceramic composition to be fused for dental prostheses made of titanium or titanium alloys, whereby the dental composition has a thermal expansion of 7.7 x 10⁻⁶ /K.

Patent Abstracts of Japan, Vol. 13 No. 523 (C-657) (3871) 1989 discloses a glare composition for artificial teeth having a coefficient of thermal expansion in the range of 40-100 x 10⁻⁷ /K (30-380°C).

The veneering or overlaying of prostheses (e.g. metallic crowns, bridge frames, or prostheses parts) is a well known and widely practiced dental technique. The metals used are almost exclusively gold alloys, palladium alloys, chromium-cobalt alloys or chromium-nickel alloys. However, more and more efforts are being taken to produce dental metal objects or restoratives out of titanium or titanium alloys. The reasons for the increased interest in titanium and titanium alloys include their bio-compatibility with the soft and hard tissues of the oral cavity and the development of new casting methods and techniques. For example, titanium and titanium alloys can be used in melting and casting procedures in a vacuum, in melting and casting procedures in an inert atmosphere, in computer-assisted milling procedures and with spark erosion techniques. Additional advantages of titanium and its alloys include low specific weight, good erosion resistance and low price compared to that for a precious or semi-precious metal.

It is well known in the dental arts that it is very difficult to produce strong bonding between titanium and available dental ceramics. One reason for this difficulty is because the firing temperature should not exceed 880°C. Above this temperature, titanium shows a significantly increased oxygen and nitrogen absorption as well as an alpha to beta structural modification. The increased absorption and structural modification lead to the embrittlement of the titanium surface where a decline in the strength of the metal takes place.

Another reason for the difficulty in producing strong bonding between titanium and available dental ceramics is because the thermal expansion of the ceramic should be adjusted quite exactly to the thermal expansion of the titanium. Laboratory experience has shown the desired range for the coefficient of thermal expansion of the ceramic to be fused to the titanium to be between 7.8 x 10⁻⁶/K to 9.0 x 10⁻⁶/K (25-500°C). Additionally, the dental ceramic should wet the titanium surface so that a ceramic-titanium bond of sufficient strength is achieved.

Throughout this application, the term dental ceramic is defined as meaning any ceramic having a glassy and/or crystalline phase.

In the dental arts, experts agree that a bonding strength of 20 Megapascals (MPa) between metal and ceramic should be attained so that the resulting construction will be usable in the oral cavity for many years. The bonding between dental ceramics and the customary alloys described above results in bonding values between 22 and 35 MPa. The dental ceramics so far offered on the market for bonding with titanium show bonding strengths around 15 MPa (see Table I below).

In addition to the above-discussed functional aspects, esthetic requirements must also be considered with the use of dental ceramics on titanium. The titanium or titanium alloys are preferably veneered with multilayers and/or multishades of dental ceramics in order to achieve natural appearing tooth colors and stains. Additionally, the dental ceramic may be mixed with suitable pigments, e.g. (Ti, Cr, Sb)O₂, (Ti, Cr, W)O₂, (Zn, Cr, Fe, Al)ₓO_{y}, (Zn, Cr, Fe, Al, Sn)ₓO_{y}, (Zr, Si, Pr)ₓO_{y}, (Sn, Sb, V)ₓO_{y}, and opacifiers, e.g. TiO₂, SnO₂, ZrSiO₄, ZrO₂, in order to imitate the natural tooth shades.

It is, therefore, the primary object of the present invention to prepare a dental ceramic material suitable for high bonding strength fusion to titanium or titanium alloys.

Another object of the present invention is to prepare a dental ceramic that will give a bond strength of at least 20 MPa when bonded to titanium or titanium alloys.

It is a further object of the present invention to prepare a dental ceramic material that satisfies the esthetic requirements necessary to achieve natural appearing tooth colors and stains in dental restoratives when bonded to titanium or titanium alloys.

### SUMMARY OF THE INVENTION

A dental ceramic composition is provided according to Claim 1 that can be fused with high bonding strength to dental prostheses including crowns, bridges, or prostheses parts made of titanium or titanium alloys. The ceramic has between 8 and 17 weight-percent of boron expressed as B₂O₃ and this specific range for B₂O₃ in the ceramic is directly responsible for the high bonding strength. The ceramic material may be prepared as a dental powder or as a suspension or paste and may contain pigments or opacifiers to give natural appearing tooth colors and stains in dental restoratives when bonded to substrates of titanium or titanium alloys.

Two United States patents disclose dental porcelains with B₂O₃ concentration ranges that approach or overlap those of the present invention. However, neither patent teaches the ability of the porcelain to permanently bond to titanium or titanium alloys as is taught in the present application.

Binnes et al., U.S. Patent 4,198,244, disclose a dental porcelain having a B₂O₃ concentration range of 5-10% for jacket crowns but 0-4% for metal-bonded restorations. Mabie et al., U.S. Patent 4,431,451, teach dental materials having frits with B₂O₃ concentration ranges of 0-10% (weight percent).

Both U.S. patents disclose ranges for B₂O₃ concentrations with upper limits that approach or overlap the lower ranges for the B₂O₃ concentrations of the present invention. However, neither patent teaches the ability of the porcelain to bond to titanium or titanium alloys as described in the present application.

### DESCRIPTION OF THE INVENTION AND PREFERRED EMBODIMENTS

The present invention, in one of its primary aspects, provides a dental ceramic material according to Claim 1 having between 8 and 17 weight-percent B₂O₃ that can be fused to titanium or titanium alloys with high bonding strength. This specific content range for B₂O₃ in the ceramic is directly responsible for the high bonding strength attained.

The present invention is disclosed in a dental ceramic of the following composition:

| COMPOUND | WEIGHT-PERCENT |
|---|---|
| SiO₂ | 54-64 |
| Al₂O₃ | 5-12 |
| B₂O₃ | 8-17 |
| Li₂O | 0-6 |
| Na₂O | 3-10 |
| K₂O | 0-8 |
| MgO | 0-2 |
| CaO | 0-6 |
| BaO | 0-2 |

Other components, for example, opacifiers, pigments, other elements and/or impurities, may also be present.

A preferred embodiment is disclosed in a dental ceramic of the following composition:

| COMPOUND | WEIGHT-PERCENT |
|---|---|
| SiO₂ | 58-64 |
| Al₂O₃ | 6-12 |
| B₂O₃ | 10-13 |
| Li₂O | 2-5 |
| Na₂O | 4-7 |
| K₂O | 4-8 |
| CaO | 3-5 |

The preferred embodiment is especially suited for a high bonding strength with titanium. The thermal expansion of titanium and ceramic are particularly well adjusted to each other in this embodiment. The preferred firing temperature is preferably between 740° and 850°C, more preferably between 770° and 800°C, and most preferably at 790°C.

As previously mentioned, the dental ceramic may be mixed with suitable pigments e.g. (Ti, Cr, Sb)O₂, (Ti, Cr, W)O₂, (Zn, Cr, Fe, Al)ₓO_{y}, (Zn, Cr, Fe, Al, Sn)ₓO_{y}, (Zr, Si, Pr)ₓO_{y}, (Sn, Sb, V)ₓO_{y}, and opacifiers, e.g., TiO₂, SnO₂, ZrO₂, ZrSiO₄, in order to imitate the natural tooth shades. In this regard, a preferred embodiment of the dental ceramic containing pigments and opacifiers is the following composition:

| COMPOUND | WEIGHT-PERCENT |
|---|---|
| SiO₂ | 54-64 |
| Al₂O₃ | 5-12 |
| B₂O₃ | 8-17 |
| Li₂O | 0-6 |
| Na₂O | 3-10 |
| K₂O | 0-8 |
| MgO | 0-2 |
| CaO | 0-6 |
| BaO | 0-2 |
| TiO₂ | 0-10 |
| SnO₂ | 0-10 |
| ZrO₂ | 0-10 |
| Pigments | 0-10 |

In a preferred embodiment of the dental ceramic containing pigments and opacifiers, SnO₂ at 5% is used as an opacifier along with 2% pigments.

The dental ceramic can be prepared as a powder or as a suspension or paste in suitable organic and/or inorganic liquids. If the suspension or paste contains polymerizable compounds (monomers or prepolymers) the suspension or paste can be self-cured or cured by visible light. When a suspension or paste is fired, any organic parts should be completely burned out in usual practice.

More specifically, one use of the dental ceramic comprises mixing the dental ceramic powder with an aqueous vehicle to provide a slurry of ceramic, supplying the slurry of ceramic to the metal substrate to build-up the desired shape and thickness, and firing the ceramic by procedures well known in the art.

An alternative use of the dental ceramic comprises mixing the dental ceramic powder with a curable resin modeling liquid vehicle to prepare a suspension or spreadable paste, applying the paste to a dental restorative to provide a coating of ceramic material thereon, curing the resin to effectively fix the coating in its desired shape and thickness, and firing the dental restorative and coating to fuse the ceramic material. A method of preparation of a curable resin modeling liquid and the use of the liquid with a dental ceramic are presented in U.S. Patent Application Serial No. 07/157,206.

The polymerizable resin modeling liquid used may be a one-component or multi-component light curable resin or a self-cured (chemically-cured) multi-component resin. In the firing step, the dental restorative is fired at temperatures effective to vaporize the volatile components of the resin without burning, charring or boiling and then to fuse the ceramic material.

This invention is further illustrated by the following Examples and Table:

### EXAMPLE 1

A dental ceramic was prepared by mixing the following raw materials in a share mixer:

| RAW MATERIAL | SUPPLIER | WEIGHT-PERCENT |
|---|---|---|
| Feldspar¹ | Dentsply International, York, PA | 29 |
| Quartz Flour | Bremthaler Quarzwerke, Usingen | 35 |
| KNO₃ | Riedel-de Haen, Seelze (Art. No. 12651) | 6 |
| Na₂CO₃ | Emesco, Frankfurt | 5 |
| Li₂CO₃ | Merck, Darmstadt (Art. No. 5670) | 6 |
| CaCO₃ | Schäfer Kreice | 6 |
| Na₂B₄O₇.1OH₂O | Brenntag AG, Mühlheim/Ruhr | 6 |
| B₂O₃ | Riedel-de Haen, Seelze (Art. No. 31145) | 7 |

| | | |
|---|---|---|
| ¹ dental grade potash feldspar as described in U.S. Patent No. 4,604,366. | | |

After mixing, the blend was continuously fired in a furnace at 1450°C for 20 minutes. The resulting enamel, in the form of a frit, was chilled in water and the dry frit ground in a ball mill until 50% of the grain size was 20 microns or less. The resulting dental ceramic powder had the following composition:

| COMPOUND | WEIGHT-PERCENT |
|---|---|
| SiO₂ | 63.3 |
| Al₂O₃ | 6.4 |
| B₂O₃ | 10.8 |
| Li₂O | 2.8 |
| Na₂O | 5.7 |
| K₂O | 7.0 |
| CaO | 4.0 |

The dental ceramic powder was then mixed with water to provide a ceramic slurry. The slurry was applied to a metal substrate with a brush and the substrate and slurry fired in a furnace at 790°C for 3 minutes. Of the 3 minutes in the furnace, 2.6 minutes were under vacuum (≦ 50 mbar).

### EXAMPLE 2

A dental ceramic was prepared as in Example 1 by mixing the following raw materials:

| RAW MATERIAL | SUPPLIER | WEIGHT-PERCENT |
|---|---|---|
| Feldspar K 31 | Mandt, Gmbh & Co. KG, Brake | 51.0 |
| Quartz Flour | Bremthaler Quarzwerke, Usingen | 20.0 |
| K₂CO₃ | Merck, Darmstadt (Art. No. 4924) | 7.3 |
| Li₂CO₃ | Merck, Darmstadt (Art. No. 5670) | 4.4 |
| CaCO₃ | Merck, Darmstadt (Art. No. 2069) | 6.3 |
| B₂O₃ | Riedel-de Haen, Seelze (Art. No. 31145) | 11.0 |

The resulting dental ceramic powder had the following composition:

| COMPOUND | WEIGHT-PERCENT |
|---|---|
| SiO₂ | 60.4 |
| Al₂O₃ | 10.2 |
| B₂O₃ | 11.9 |
| Li₂O | 1.9 |
| Na₂O | 5.8 |
| K₂O | 5.8 |
| CaO | 4.0 |

The dental ceramic was subjected to a firing temperature of between 800° and 840°C and found to have a coefficient of thermal expansion of 8.0 x 10⁻⁶/K (25-500°C) and a resultant bond strength of 24 ± 2 MPa. The bond strength was determined according to the Schmitz/Schulmeyer method (K. H. Schmitz and H. Schulmeyer, Determination of bonding strength of dental-metal ceramic bonding systems, Dental-Labor, 23:1416-1420, 1975).

### EXAMPLE 3

A dental ceramic containing an opacifier was prepared as in Example 1 by mixing the following raw materials:

| RAW MATERIAL | SUPPLIER | WEIGHT-PERCENT |
|---|---|---|
| Feldspar | Dentsply International, York, PA | 47.0 |
| Quartz Flour | Bremthaler Quarzwerke, Usingen | 22.0 |
| Na₂CO₃ | Emesco, Frankfurt | 4.0 |
| Li₂CO₃ | Merck, Darmstadt (Art. No. 5670) | 7.0 |
| CaCO₃ | Merck, Darmstadt (Art. No. 2069) | 6.0 |
| TiO₂, RN56 | Kronos Titan GmbH, Leverkusen | 2.0 |
| B₂O₃ | Riedel-de Haen, Seelze (Art. No. 31145) | 6.0 |
| Na₂B₄O₇.10H₂O | Brenntag AG, Mühlheim/Rohr | 6.0 |

The resulting dental ceramic containing an opacifier had the following composition:

| COMPOUND | WEIGHT-PERCENT |
|---|---|
| SiO₂ | 60.0 |
| Al₂O₃ | 10.0 |
| B₂O₃ | 9.3 |
| TiO₂ | 2.3 |
| Li₂O | 3.2 |
| Na₂O | 5.5 |
| K₂O | 5.9 |
| CaO | 3.9 |

Again, the ceramic was subjected to a firing temperature of between 800° and 840°C and found to have a coefficient of thermal expansion of 8.0 x 10⁻⁶/K (25-500°C). As in Example 2, the resultant bond strength was determined according to the Schmitz/Schulmeyer method to be 24 ± 2 MPa.

Additional determinations of bonding strength between pure titanium and ceramic materials were performed and the results presented in Table I. Samples I-IV are ceramic materials of varying B₂O₃ content. Duceratin and Titanbond are commercially available ceramics for bonding to titanium. Duceratin is the product of Ducera Dental GmbH, Rodheimer Str. 7, D-6365 Rosbach v.d.H., West Germany and Titanbond is the product of Asami Tanaka Dental Enterprises Europe GmbH, Kaiser-Friedrich-Promenade 26, D-6380 Bad Homburg, v.d.H., West Germany. The bonding strength was determined according to the Schmitz/Schulmeyer method.

Samples II and III are preferred embodiments. Sample II is the same as Example 2. Samples I and IV which are respectively below and above the claimed B₂O₃ content demonstrate lower bonding strength.

**TABLE I**

| | Ti | I | II | III | IV | Duceratin | Titanbond |
|---|---|---|---|---|---|---|---|
| Bonding strength [MPa] | - | 14± 2 | 24± 2 | 20± 2 | 17± 2 | 15± 2 | 12± 2 |
| Thermal expansion* [ 10⁻⁶/K] | 9.3 | 8.7 | 8.0 | 8.2 | 8.6 | - | - |
| Fire temperature [°C] | - | 810 | 800-840 | 820 | 790 | 750 | 800 |
| SiO₂ | | 62.2 | 60.4 | 59.4 | 52.3 | | |
| Al₂O₃ | | 10.4 | 10.2 | 10.1 | 9.6 | | |
| B₂O₃ | | 5.5 | 11.9 | 12.3 | 17.4 | | |
| TiO₂ | | 2.2 | - | - | - | | |
| Li₂O | | 1.9 | 1.9 | 1.8 | 1.9 | | |
| Na₂O | | 7.0 | 5.8 | 6.5 | 7.7 | | |
| K₂O | | 6.2 | 5.8 | 6.0 | 5.7 | | |
| CaO | | 4.6 | 4.0 | 3.9 | 2.7 | | |
| BaO | | - | - | - | 2.7 | | |

| | | | | | | | |
|---|---|---|---|---|---|---|---|
| * 25-500°C | | | | | | | |

### EXAMPLE 4

Another dental ceramic containing an opacifier was prepared as in Example 1 by mixing the following raw materials:

| RAW MATERIAL | SUPPLIER | WEIGHT-PERCENT |
|---|---|---|
| Feldspar | Dentsply International, York, PA | 28.0 |
| Quartz Flour | Bremthaler Quarzwerke, Usingen | 34.0 |
| Na₂CO₃ | Emesco, Frankfurt | 5.0 |
| KNO₃ | Riedel-de Haen, Seelze (Art. No. 12651) | 6.0 |
| Li₂CO₃ | Merck, Darmstadt (Art. No. 5670) | 6.0 |
| CaCO₃ | Merck, Darmstadt (Art. No. 2069) | 6.0 |
| B₂O₃ | Riedel-de Haen, Seelze (Art. No. 31145) | 7.0 |
| Na₂B₄O₇.10H₂O | Brenntag AG, Mühlheim/Ruhr | 6.0 |
| TiO₂, RN56 | Kronos Titan GmbH, Leverkusen | 2.0 |

The resulting dental ceramic containing an opacifier had the following composition:

| COMPOUND | WEIGHT-PERCENT |
|---|---|
| SiO₂ | 61.6 |
| Al₂O₃ | 6.4 |
| K₂O | 7.0 |
| Na₂O | 5.6 |
| Li₂O | 2.8 |
| CaO | 3.9 |
| B₂O₃ | 10.5 |
| TiO₂ | 2.2 |

The ceramic was subjected to a firing temperature of 800°C and had a coefficient of thermal expansion of 8.6 x 10⁻⁶/K (25-500°C). The resultant bond strength was determined to be 22 ± 2 MPa according to the Schmitz/Schulmeyer method.

## Claims

1. A dental ceramic material that can be fused to dental protheses made of titanium or titanium alloys, comprising:
| COMPOUND | WEIGHT-PERCENT |
|---|---|
| SiO₂ | 54-64 |
| Al₂O₃ | 5-12 |
| B₂O₃ | 8-17 |
| Li₂O | 0-6 |
| Na₂O | 3-10 |
| K₂O | 0-8 |
| MgO | 0-2 |
| CaO | 0-6 |
| BaO | 0-2 |
wherein said material has a coefficient of thermal expansion of 7.8 x 10⁻⁶/K to 9.0 x 10⁻⁶/K (25-500°C) and can be fused to titanium or titanium alloys at temperatures between 740° and 850°C.

2. The dental ceramic material of claim 1 wherein said B₂O₃ is between 10-13 weight-percent.

3. The dental ceramic material of claim 1 further comprising polymerizable compounds, opacifiers and/or pigments and wherein said dental ceramic material is a powder suspension or spreadable paste which is self-curable or curable by visible light.

4. The dental ceramic material of claim 2 further comprising organic compounds that are completely burned out upon firing of said dental ceramic material to said titanium or titanium alloys

5. A method for preparing dental protheses from titanium or titanium alloys bonded with a dental ceramic material, comprising the steps of
a) preparing a suspension or spreadable paste from said dental ceramic material, said dental ceramic having a composition as defined in claim 1,
b) applying said suspension or spreadable paste to a formed structure,
c) firing said suspension or spreadable paste.

6. The method of claim 5 wherein said B₂O₃ is between 10-13 weight-percent.

7. The method of claim 5 further comprising polymerizable compounds, opacifiers and/or pigments and wherein said dental ceramic material is a powder, suspension or spreadable paste which is self-curable or curable by visible light.

8. The method of claim 5 wherein organic compounds are completly burned out upon firing of said dental ceramic material to said titanium or titanium alloys and wherein said material is fused to titanium or titanium alloys at temperatures between 740° and 850°C.

## Patentansprüche

1. Dentales keramisches Material, das an Zahnprothesen, die aus Titan oder Titanlegierungen hergestellt sind, geschmolzen werden kann, umfassend:
| VERBINDUNG | GEWICHTSPROZENT |
|---|---|
| SiO₂ | 54-64 |
| Al₂O₃ | 5-12 |
| B₂O₃ | 8-17 |
| Li₂O | 0-6 |
| Na₂O | 3-10 |
| K₂O | 0-8 |
| MgO | 0-2 |
| CaO | 0-6 |
| BaO | 0-2 |
wobei das Material einen thermischen Ausdehnungskoeffizienten von 7,8 x 10⁻⁶/K bis 9,0 x 10⁻⁶/K (25-500°C) aufweist und an Titan oder Titanlegierungen bei Temperaturen zwischen 740° und 850°C geschmolzen werden kann.

2. Dentales keramisches Material nach Anspruch 1, wobei das B₂O₃ zwischen 10-13 Gewichtsprozent beträgt.

3. Dentales keramisches Material nach Anspruch 1, das außerdem polymerisierbare Verbindungen, Trübungsmittel und/oder Pigmente umfaßt, und wobei das dentale keramische Material eine Pulversuspension oder eine verstreichbare Paste darstellt, die selbsthärtbar oder durch sichtbares Licht härtbar ist.

4. Dentales keramisches Material nach Anspruch 2, das außerdem organische Verbindungen umfaßt, die vollständig nach Brennen des dentalen keramischen Materials an das Titan oder die Titanlegierungen aufgebraucht sind.

5. Verfahren zur Herstellung von Zahnprothesen aus Titan oder Titanlegierungen, die mit einem dentalen keramischen Material verbunden sind, umfassend die Schritte
a) Herstellen einer Suspension oder verstreichbaren Paste aus dem dentalen keramischen Material, wobei die dentale Keramik eine wie in Anspruch 1 definierte Zusammensetzung aufweist,
b) Auftragen der Suspension oder verstreichbaren Paste auf eine geformte Struktur,
c) Brennen der Suspension oder verstreichbaren Paste.

6. Verfahren nach Anspruch 5, wobei das B₂O₃ zwischen 10-13 Gewichtsprozent ausmacht.

7. Verfahren nach Anspruch 5, das außerdem polymerisierbare Verbindungen, Trübungsmittel und/oder Pigmente umfaßt, und wobei das dentale keramische Material eine Pulversuspension oder eine verstreichbare Paste darstellt, die selbsthärtbar oder durch sichtbares Licht härtbar ist.

8. Verfahren nach Anspruch 5, wobei die organischen Verbindungen vollständig nach Brennen des dentalen keramischen Materials an das Titan oder die Titanlegierungen aufgebraucht sind und wobei das Material an Titan oder Titanlegierungen bei Temperaturen zwischen 740° und 850°C geschmolzen wird.

## Revendications

1. Matière de céramique dentaire qui peut être fusionnée à des prothèses dentaires faites de titane ou d'alliages de titane, comprenant :
| COMPOSE | POURCENTAGE EN MASSE |
|---|---|
| SiO₂ | 54 à 64 |
| Al₂O₃ | 5 à 12 |
| B₂O₃ | 8 à 17 |
| Li₂O | 0 à 6 |
| Na₂O | 3 à 10 |
| K₂O | 0 à 8 |
| MgO | 0 à 2 |
| CaO | 0 à 6 |
| BaO | 0 à 2 |
dans laquelle ladite matière a un coefficient de dilatation thermique de 7,8 x 10⁻⁶/K à 9,0 x 10⁻⁶/K (25 à 500°C) et peut être fusionnée avec du titane ou avec des alliages de titane à des températures se situant entre 740°C et 850°C.

2. Matière de céramique dentaire selon la revendication 1, dans laquelle ledit B₂O₃ se trouve en un pourcentage, en masse, entre 10 et 13 %.

3. Matière de céramique dentaire selon la revendication 1, comprenant de plus des composés polymérisables, des opaqueurs et/ou des pigments et dans laquelle ladite matière de céramique dentaire est une suspension de poudre ou une pâte, pouvant être étalée, qui est autopolymérisable ou polymérisable par lumière visible.

4. Matière de céramique dentaire selon la revendication 2, comprenant de plus des composés organiques qui sont complètement éliminés par brûlage, au moment de la cuisson de ladite matière de céramique dentaire avec ledit titane ou avec lesdits alliages de titane.

5. Procédé pour préparer les prothèses dentaires à partir de titane ou d'alliages de titane liés avec une matière de céramique dentaire comprenant les étapes :
a) de préparation d'une suspension ou pâte pouvant être étalée à partir de ladite matière de céramique dentaire, ladite céramique dentaire ayant une composition comme définie dans la revendication 1 ;
b) d'application de ladite suspension ou pâte pouvant être étalée sur une structure formée ;
c) de cuisson de ladite suspension ou pâte pouvant être étalée.

6. Procédé selon la revendication 5, dans lequel ledit B₂O₃ se trouve en pourcentage, en masse, entre 10 et 13 %.

7. Procédé selon la revendication 5, comprenant de plus les composés polymérisables, les opaqueurs et/ou des pigments et dans lequel ladite matière de céramique dentaire est une poudre, une suspension ou pâte pouvant être étalée, qui est auto-polymérisable ou polymérisable par la lumière visible.

8. Procédé selon la revendication 5, dans lequel les composés organiques sont complètement éliminés par brûlage, au moment de la cuisson de ladite matière de céramique dentaire avec ledit titane ou avec lesdits alliages de titane et dans lequel ladite matière est fusionnée avec le titane ou avec les alliages de titane à des températures situées entre 740°C et 850°C.
